# EUROPÄISCHE PATENTSCHRIFT

(11) **EP 1 572 706 B1**
(45) Veröffentlichungstag und Bekanntmachung des Hinweises auf die Patenterteilung: **15.03.2006**
(21) Anmeldenummer: 03785775.2
(22) Anmeldetag: 09.12.2003
(51) Int. Cl.: C07H 15/04

(54) **VERFAHREN ZUR HERSTELLUNG VON ALKYL- UND/ODER ALKENYLOLIGLYKOSIDCARBONS URE-SALZEN**
METHOD FOR PRODUCING ALKYL OLIGOGLUCOSIDE- AND/OR ALKENYL OLIGOGLUCOSIDE CARBOXYLIC ACID SALTS
PROCEDE DE PRODUCTION DE SELS D'ACIDE ALKYLOLIGOGLYCOSIDE-CARBOXYLIQUE ET/OU ALCENYLOLIGOGLYCOSIDE-CARBOXYLIQUE

(30) Priorität: 19.12.2002 DE 10259403
(43) Veröffentlichungstag der Anmeldung: 14.09.2005
(73) Patentinhaber: Cognis IP Management GmbH, 40589 Düsseldorf (DE)
(72) Erfinder: BEHLER, Ansgar, 46240 Bottrop (DE); FOLGE, Almud, 42799 Leichlingen (DE)
(74) Vertreter: Gittinger, Andreas
(86) Internationale Anmeldenummer: PCT/EP2003/013920
(87) Internationale Veröffentlichungsnummer: WO 2004/056842

(56) Entgegenhaltungen:
- WO-A-97/42299
- WO-A-02/090369

## Beschreibung

### Gebiet der Erfindung

Die Erfindung befindet sich auf dem Gebiet der oberflächenaktiven Verbindungen und betrifft ein neues Verfahren zur Herstellung von speziellen Aniontensiden, welche sich durch einen verminderten Gehalt an organischen Chlorverbindungen auszeichnen.

### Stand der Technik

Carboxylierungsprodukte von Alk(en)yloligoglykosiden stellen anionische Tenside dar, die gegenüber den nicht-derivatisierten Homologen über verbesserte Eigenschaften im Bereich der Schaumbildung, Viskosität und Sensorik verfügen. Üblicherweise erfolgt ihre Herstellung durch Umsetzung der Glykoside mit Halogencarbonsäuresalzen, speziell Natriumchloracetat in wässriger Lösung. In Abhängigkeit der Menge des eingesetzten Alkylierungsmittels werden einige oder alle der zur Verfügung stehenden Hydroxylgruppen umgesetzt. Da diese Reaktion in der Regel einen Überschuss des Alkylierungsmittels erfordert, finden sich in den Endprodukten stets noch Spuren organischer Chlorverbindungen, die bezogen beispielsweise auf Monochloracetat bis zu 2.000 ppm und auf das im Monochloracetat als Verunreinigung enthaltene Dichloracetat bis zu 500 ppm betragen können.

Für eine Anwendung insbesondere im Kosmetikbereich sind solche Mengen an Organochlorverbindungen nicht akzeptabel, da sie Hautirritationen verursachen können oder in sonstiger Weise aus physiologischen Gründen unerwünscht sind.

Die Aufgabe der vorliegenden Erfindung hat somit darin bestanden, ein verbessertes Verfahren zur Herstellung von Alk(en)yloligoglykosidcarbonsäure-Salzen zur Verfügung zu stellen, welches Produkte liefert, deren Gehalt an Organochlorverbindungen unter 35 ppm und vorzugsweise unter 10 ppm liegt.

### Beschreibung der Erfindung

Gegenstand der Erfindung ist ein neues Verfahren zur Herstellung von wässrigen Pasten von Alkyl- und/oder Alkenyloligoglykosidcarbonsäure-Salzen mit vermindertem Gehalt an organischen Chlorverbindungen, welches sich dadurch auszeichnet, dass man die Alkyl- und/oder Alkenyloligoglykoside in an sich bekannter Weise mit Halogencarbonsäuren oder deren Salzen umsetzt und die Reaktionsprodukte bei Temperaturen im Bereich von 50 bis 120 °C einer alkalischen Nachbehandlung unterwirft.

Überraschenderweise wurde gefunden, dass durch alkalische Nachbehandlung der Pasten der Gehalt an organischen Chlorverbindungen in Summe mindestens unter 35 ppm, in der Regel sogar unter 10 ppm gesenkt werden kann.

### Alkyl- und/oder Alkenyloligoglykoside

Alkyl- und Alkenyloligoglykoside, die als eine der beiden Ausgangsstoffe für die Herstellung der Carbonsäuresalze in Frage kommen, stellen bekannte nichtionische Tenside dar, die der Formel (I) folgen,

**R**^{**1**}**O-[G]**_{**p**} **(I)**

in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligoglucoside. Die Indexzahl p in der allgemeinen Formel (I) gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muss und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt.

Der Alkyl- bzw. Alkenylrest R¹ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestem oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R¹ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 1,8.

### Halogencarbonsäuren

Zur Einführung der Carboxylfunktionen in die Glykoside werden Halogencarbonsäuren oder deren Salze eingesetzt, die vorzugsweise der Formel (II) folgen,

**Cl(CH**_{**2**}**)**_{**n**}**COOX** **(II)**

in der n für Zahlen von 1 bis 5 und X für Wasserstoff oder ein Alkalimetall, vorzugsweise Natrium oder Kalium steht. Insbesondere werden Chloressigsäure oder deren Natriumsalz verwendet.

### Durchführung des Verfahrens

Die Carboxylierung der Alk(en)yloligoglykoside kann in an sich bekannter Weise, d.h. durch direkte Umsetzung der Reaktionspartner bei erhöhter Temperatur in wässriger Lösung durchgeführt werden. Dabei setzt man die Alkyl- und/oder Alkenyloligoglykoside und die Halogencarbonsäuren bzw. deren Salze typischerweise im Molverhältnis 1 : 0,9 bis 1 : 5, vorzugsweise 1 : 1,05 bis 1 : 3 und insbesondere 1 : 1,2 bis 1 : 2 ein. Der Überschuss an Halogencarbonsäuren richtet sich dabei in erster Linie nach der Anzahl der Carboxylfunktionen, die auf diese Weise in das Molekül eingeführt werden sollen. Auf diese Weise werden wässrige Pasten von Alkyl- und/oder Alkenyloligoglykosidcarbonsäure-Salzen einsetzt, die einen Feststoffgehalt von 30 bis 60 und insbesondere 40 bis 50 Gew.-% sowie Gehalte an organischen Chlorverbindungen von bis zu 2.500 ppm aufweisen. Zu deren Abbau werden die Pasten einer alkalischen Nachbehandlung unterworfen, d.h. durch Zugabe von wässrigen Alkaliverbindungen, vorzugsweise Natron- oder Kalilauge auf einen pH-Wert von 10 bis 14 und vorzugsweise 11 bis 13 eingestellt. Überraschend hat sich erwiesen, dass die Glykosidcarbonsäure-Salze unter diesen Bedingungen keine Hydrolyse erleiden. Als besonders vorteilhaft hat sich eine Nachbehandlung - gegebenenfalls unter (autogenem) Druck ― bei einer Temperatur von 60 bis 110°C, und insbesondere 70 bis 90 °C erwiesen. Weiterhin empfehlenswert ist es, die Nachbehandlung unter Ausschluss von Luftsauerstoff, also unter Inertgasabdeckung durchzuführen, um eine Verfärbung der Produkte zu verhindern. Die Reaktionszeit ergibt sich aus dem Zusammenwirken von pH-Wert und Temperatur und liegt typisch bei 2 bis 5 Stunden. Tatsächlich empfiehlt es sich, den Verlauf der Nachbehandlung durch Probennahme zu verfolgen und die Reaktion abzubrechen, sobald der Gehalt an Monochlorverbindungen unter 5 ppm und an Dichlorverbindungen unter 30 ppm gesunken ist.

### Beispiele

### Beispiel 1

In einem Rührbehälter wurden 550 g einer 50 Gew.-%igen wässrigen Paste eines C_{12/14-}Kokosalkyloligoglucosidcarboxylats (Plantapon® LGC, Cognis Deutschland GmbH & Co. KG) mit einem Restgehalt von 314 ppm Monochloracetat und 58 ppm Dichloracetat vorgelegt und mit 13,7 g wässriger 50 Gew.-%iger Natriumhydroxidlösung auf einen pH-Wert von 13,5 (gemessen in 10 Gew.-%iger Verdünnung) eingestellt. Anschließend wurde die Mischung unter einer Stickstoffabdeckung bei einer Temperatur von 90 bis 95 °C über einen Zeitraum von 3 h gerührt. Das gereinigte Produkt wurde als gellgelbe Flüssigkeit erhalten und wies nach der Behandlung noch einen Monochloracetatgehalt von weniger als 1 ppm und einer Dichloracetatgehalt von weniger als 5 ppm auf.

### Beispiel 2

In einem Rührbehälter wurden 550 g einer 50 Gew.-%igen wässrigen Paste eines C_{12/14-}Kokosalkyloligoglucosidcarboxylats (Plantapon® LGC, Cognis Deutschland GmbH & Co. KG) mit einem Restgehalt von 314 ppm Monochloracetat und 58 ppm Dichloracetat vorgelegt und mit 10,4 g wässriger 50 Gew.-%iger Kaliumhydroxidlösung auf einen pH-Wert von 13,5 (gemessen in 10 Gew.-%iger Verdünnung) eingestellt. Anschließend wurde die Mischung unter einer Stickstoffabdeckung bei einer Temperatur von 90 bis 95 °C über einen Zeitraum von 2,5 h gerührt. Das gereinigte Produkt wurde als gellgelbe Flüssigkeit erhalten und wies nach der Behandlung noch einen Monochloracetatgehalt von weniger als 1 ppm und einer Dichloracetatgehalt von weniger als 5 ppm auf.

## Patentansprüche

1. Verfahren zur Herstellung von wässrigen Pasten von Alkyl- und/oder Alkenyloligoglykosidcarbonsäure-Salzen mit vermindertem Gehalt an organischen Chlorverbindungen, **dadurch gekennzeichnet, dass** man die Alkyl- und/oder Alkenyloligoglykoside in an sich bekannter Weise mit Halogencarbonsäuren oder deren Salzen umsetzt und die Reaktionsprodukte bei Temperaturen im Bereich von 50 bis 120 °C einer alkalischen Nachbehandlung unterwirft.

2. Verfahren nach Anspruch 2, **dadurch gekennzeichnet, dass** man Alkyl- und/oder Alkenyloligoglykoside der Formel (I) einsetzt,
**R**^{**1**}**O-[G]**_{**p**} **(I)**
in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht.

3. Verfahren nach den Ansprüchen 1 und/oder 2, **dadurch gekennzeichnet, dass** man Alkylglucoside der Formel (**I**) einsetzt, in der R¹ für einen Alkylrest mit 12 bis 18 Kohlenstoffatomen, G für eine Glucoseeinheit und p für Zahlen von 1 bis 1,8 steht.

4. Verfahren nach mindestens einem der Ansprüche 1 bis 3, **dadurch gekennzeichnet, dass** man Halogencarbonsäuren oder deren Salze der Formel (II) einsetzt,
**Cl(CH**_{**2**}**)**_{**n**}**COOX** **(II)**
in der n für Zahlen von 1 bis 5 und X für Wasserstoff oder ein Alkalimetall steht.

5. Verfahren nach mindestens einem der Ansprüche 1 bis 4, **dadurch gekennzeichnet, dass** man als Alkylierungsmittel Chloressigsäure-oder deren Natriumsalz einsetzt.

6. Verfahren nach mindestens einem der Ansprüche 1 bis 5, **dadurch gekennzeichnet, dass** man die Alkyl- und/oder Alkenyloligoglykoside und die Halogencarbonsäuren bzw, deren Salze im Molverhältnis 1 : 0,9 bis 1 : 5 einsetzt.

7. Verfahren nach mindestens einem der Ansprüche 1 bis 6, **dadurch gekennzeichnet, dass** man wässrige Pasten von Alkyl- und/oder Alkenyloligoglykosidcarbonsäure-Salzen einsetzt, die einen Feststoffgehalt von 30 bis 60 Gew.-% aufweisen.

8. Verfahren nach mindestens einem der Ansprüche 1 bis 7, **dadurch gekennzeichnet, dass** man die wässrigen Pasten durch Zugabe von wässrigen Alkaliverbindungen auf einen pH-Wert von 10 bis 14 einstellt.

9. Verfahren nach mindestens einem der Ansprüche 1 bis 9, **dadurch gekennzeichnet, dass** man die wässrigen Pasten bei einer Temperatur von 70 bis 90 °C nachbehandelt.

10. Verfahren nach mindestens einem der Ansprüche 1 bis 10, **dadurch gekennzeichnet, dass** man die wässrigen Pasten solange nachbehandelt, bis der Gehalt an Monochlorverbindungen unter 5 ppm und an Dichlorverbindungen unter 30 ppm gesunken ist.

## Claims

1. A process for the production of water-containing pastes of alkyl and/or alkenyl oligoglycoside carboxylic acid salts with a reduced content of organochlorine compounds, **characterized in that** the alkyl and/or alkenyl oligoglycosides are reacted in known manner with halocarboxylic acids or salts thereof and the reaction products are subjected to an alkaline aftertreatment at temperatures in the range from 50 to 120°C.

2. A process as claimed in claim 2, **characterized in that** alkyl and/or alkenyl oligoglycosides corresponding to formula (I):
**R**^{**1**}**O-[G]**_{**p**} **(I)**
where R¹ is an alkyl and/or alkenyl group containing 4 to 22 carbon atoms, G is a sugar unit containing 5 or 6 carbon atoms and p is a number of 1 to 10, are used.

3. A process as claimed in claim(s) 1 and/or 2, **characterized in that** alkyl glucosides corresponding to formula (I), in which R¹ is a C₁₂₋₁₈ alkyl group, G is a glucose unit and p is a number of 1 to 1.8, are used.

4. A process as claimed in at least one of claims 1 to 3, **characterized in that** halocarboxylic acids or salts thereof corresponding to formula (II):
**Cl(CH**_{**2**}**)**_{**n**}**COOX** **(II)**
in which n is a number of 1 to 5 and X is hydrogen or an alkali metal, are used.

5. A process as claimed in at least one of claims 1 to 4, **characterized in that** chloroacetic acid or its sodium salt is used as the alkylating agent.

6. A process as claimed in at least one of claims 1 to 5, **characterized in that** the alkyl and/or alkenyl oligoglycosides and the halocarboxylic acids or their salts are used in a molar ratio of 1:0.9 to 1:5.

7. A process as claimed in at least one of claims 1 to 6, **characterized in that** water-containing pastes of alkyl and/or alkenyl oligoglycoside carboxylic acid salts with a solids content of 30 to 60% by weight are used.

8. A process as claimed in at least one of claims 1 to 7, **characterized in that** the water-containing pastes are adjusted to a pH of 10 to 14 by addition of aqueous alkali metal compounds.

9. A process as claimed in at least one of claims 1 to 9, **characterized in that** the water-containing pastes are aftertreated at a temperature of 70 to 90°C.

10. A process as claimed in at least one of claims 1 to 10, **characterized in that** the water-containing pastes are treated until the content of monochlorine compounds has fallen to below 5 ppm and the content of dichlorine compounds is below 30 ppm.

## Revendications

1. Procédé de préparation de pâtes aqueuses de sels d'acides carboxyliques d'oligoglycosides d'alkyle et/ou d'alcényle ayant une teneur réduite en composés chlorés organiques,
**caractérisé en ce qu'**
on fait réagir les oligoglycosides d'alkyle et/ou d'alcényle d'une manière connue en soi avec des acides carboxyliques halogénés ou leurs sels et on soumet les produits de la réaction à un post-traitement alcalin à des températures de l'ordre de 50 à 120°C.

2. Procédé selon la revendication 2,
**caractérisé en ce qu'**
on utilise des oligoglycosides d'alkyle et/ou d'alcényle de formule (I)
R¹O-[G]ₚ (I)
dans laquelle R¹ représente un radical alkyle et/ou alcényle portant 4 à 22 atomes de carbone, G un radical sucre portant 5 ou 6 atomes de carbone, et p un nombre de 1 à 10.

3. Procédé selon les revendications 1 et/ou 2,
**caractérisé en ce qu'**
on utilise des glucosides d'alkyle de formule (I) dans laquelle R¹ représente un radical alkyle portant 12 à 18 atomes de carbone, G une unité glucose et p un nombre de 1 à 1,8.

4. Procédé selon au moins l'une quelconque des revendications 1 à 3,
**caractérisé en ce qu'**
on utilise des acides carboxyliques halogénés ou leurs sels de formule (II)
Cl(CH₂)ₙCOOX (II)
dans laquelle n représente un nombre de 1 à 5 et X un atome d'hydrogène ou un métal alcalin.

5. Procédé selon au moins l'une quelconque des revendications 1 à 4,
**caractérisé en ce que**
comme agent d'alkylation, on utilise de l'acide chloracétique ou son sel de sodium.

6. Procédé selon au moins l'une quelconque des revendications 1 à 5,
**caractérisé en ce qu'**
on utilise les oligoglycosides d'alkyle et/ou d'alcényle et les acides carboxyliques halogénés ou leurs sels dans un rapport molaire de 1:0,9 à 1:5.

7. Procédé selon au moins l'une quelconque des revendications 1 à 6,
**caractérisé en ce qu'**
on utilise des pâtes aqueuses de sels d'acides carboxyliques d'oligoglycosides d'alkyle et/ou d'alcényle, qui présentent une teneur en substances solides de 30 à 60 % en poids.

8. Procédé selon au moins l'une quelconque des revendications 1 à 7,
**caractérisé en ce qu'**
on ajuste le pH des pâtes aqueuses à 10 à 14 en ajoutant des composés alcalins aqueux.

9. Procédé selon au moins l'une quelconque des revendications 1 à 9,
**caractérisé en ce qu'**
on soumet les pâtes aqueuses à un post traitement à une température de 70 à 90°C.

10. Procédé selon l'une quelconque des revendications 1 à 10,
**caractérisé en ce qu'**
on poursuit le post-traitement des pâtes aqueuses jusqu'à ce que leur teneur en composés monochlorés soit descendue en dessous de 5 ppm et leur teneur en composés dichlorés en dessous de 30 ppm.
